# EUROPEAN PATENT APPLICATION

(11) **EP 1 757 577 A1**
(43) Date of publication of application: **28.02.2007**
(21) Application number: 05730627.6
(22) Date of filing: 15.04.2005
(51) Int. Cl.: C07C 67/54, C07C 67/08, C07C 69/653, C08F 20/22

(54) **PROCESS FOR PRODUCING FLUORINATED ACRYLIC ESTER**

(30) Priority: 26.04.2004 JP 2004129720
(71) Applicant: DAIKIN INDUSTRIES, LTD., Osaka-shi, Osaka 566-8585 (JP)
(72) Inventor: FUNAKOSHI, Yoshirou Yodogawa Works of DAIKIN, Settsu-shi, Osaka 5668585 (JP); HIRASAKA, Takeomi, DAIKIN INDUSTRIES LTD., Settsu-shi, Osaka 5668585 (JP); TANAKA, Yoshinori, DAIKIN INDUSTRIES LTD., Settsu-shi, Osaka 5668585 (JP)
(74) Representative: HOFFMANN EITLE
(86) International application number: PCT/JP2005/007314
(87) International publication number: WO 2005/102983

(57) **Abstract**

A mixture of fluoroalkyl alcohols represented by C₂F₅(CF₂CF₂)ₙCH₂CH₂OH wherein "n" is an integer equal to or more than 0 wherein the sum of the fluoroalkyl alcohols of n=3 and n=4 contained is 85 mol% or more is prepared and reacted with an acrylic acid compound followed by removing unreacted C₂F₅(CF₂CF₂)ₙCH₂CH₂OH, to give a mixture of fluorine-containing acrylic esters wherein the content of impurities (that is, olefins represented by C₂F₅(CF₂CF₂)ₙCH=CH₂ and the alcohols represented by C₂F₅(CF₂CF₂)ₙCH₂CH₂OH) is low.

## Description

### Technical Field

The present invention is related to a method for producing a mixture of fluorine-containing acrylic esters which includes less impurities.

### Background Art

A fluorine-containing acrylic ester represented by a general formula (1):

C₂F₅(CF₂CF₂)ₙCH₂CH₂OCOCR¹=CH₂ (1)

wherein R¹ is a hydrogen atom, a methyl group or a halogen atom and "n" is an integer of at least zero is used as a monomer in production of a fluorine-containing acrylate-based polymer that may be an active component for a water-and oil-repellent. Various methods for producing this monomer have been proposed.

For example, Japanese Patent Kokoku Publication No. S39-18112(1964) and Japanese Patent Kokoku Publication No. S48-30611(1973) disclose a method for reacting a fluorine-containing alkyl halide with an alkali metal salt of a carboxylic acid in a specific solvent. Japanese Patent Kokoku Publication Nos. H4-16451(1992), H4-16452(1992) and S61-57813(1986) disclose a method for producing the fluorine-containing ester and mention that the resultant fluorine-containing ester may be converted to an acrylic ester. Further, Japanese Patent Kokai (Laid-Open) Publication No. H9-59215(1997) discloses a method for producing the fluorine-containing acrylic ester represented by the above formula (1) is produced by reacting the fluorine-containing alkyl halide with a specific betaine followed by an alkaline treatment.

Another method for producing the fluorine-containing acrylic ester is known wherein the fluoroalkyl halide is converted to the fluorine-containing alkyl alcohol that is then converted to the fluorine-containing acrylic ester. For example, in Japanese Patent Kokai (Laid-Open) Publication No. S59-181239(1984), a method for producing the fluorine-containing acrylic ester is disclosed wherein the fluorine-containing alkyl alcohol and acrylic acid or methacrylic acid are reacted under the presence of a concentrated sulfuric acid or a fuming sulfuric acid. In Patent Kokai Publication (Laid-Open) No. H2-295948(1990), a method for reacting the fluorine-containing alkyl alcohol and methacrylic acid under the presence of phosphoric anhydride is disclosed. USP No. 3,719,698 discloses a method for producing the fluorine-containing acrylic ester wherein the fluorine-containing alkyl alcohol is added to a compound that is obtained by reacting acrylic acid or methacrylic acid with a trifluoroacetic anhydride. Japanese Patent Kokai (Laid-Open) Publication Nos. S59-117503(1984), S59-117504(1984) and H3-163044(1991) disclose a method for producing the fluorine-containing acrylic ester wherein the acrylic acid halide or the methacrylic acid halide is reacted with the fluorine-containing alcohol in an aqueous solution of an alkali metal hydroxide.

In any methods, a fluoroalkyl iodide generally represented by R_{f}I (R_{f} is a fluoroalkyl group) is used as a starting material. In the method disclosed in Patent Kokoku Publication Nos. S39-18112(1964) and S48-30611(1973), an ethylene adduct of this iodide is used. In the method using the fluoroalkyl alcohol as described in Japanese Patent Kokoku Publication No. S61-57813(1986), this iodide is used as a material for the alcohol. R_{f}I is, for example, a telomer produced by a telomerization reaction.

### Disclosure of Invention

### (Problems to be Solved by Invention)

It is known that when the monomer represented by the formula (1) is produced according to the method, as described in Japanese Patent Kokoku Publication No. S61-57813(1986), wherein fluoroalkyl alcohol and the acrylic acid compound are reacted, and the monomers are polymerized, the resultant product contains, in addition to the polymer, an alcohol represented by the following formula (2):

C₂F₅(CF₂CF₂)ₙCH₂CH₂OH (2)

This alcohol does not function as the active component for the water- and oil-repellent.

Further, it is known that the preferable monomers constituting the polymer for the water- and oil-repellent are ones with "n" of at least 3 in the above formula (1). The monomers with "n" of at least 3 are produced using the fluorine-containing alcohol represented by the above formula (2) with "n" of at least 3. The monomers generally contain unreacted alcohols that remain in the polymer after the polymerization of the monomers. Of the remaining alcohols, one with "n" of three, that is, C₈F₁₇CH₂CH₂OH may be oxidized to give C₇F₁₅COOH (perfluoro octanoic acid: abbreviation PFOA). Recent research results for example, EPA report "PRELIMINARY RISK ASSESSMENT OF THE DEVELOPMENTAL TOXICITY ASSOCIATED WITH EXPOSURE TO PERFLUOROOCTANOIC ACID AND ITS SALTS" (http://www.epa.gov/opptintr/pfoa/pfoara.pdf) point out that perfluorooctanoic acid might be a burden to the environment. For this reason, the EPA (Environmental Protection Agency of US) announced, on April 14, 2003, that they would strengthen the scientific research as to PFOA.

The compound represented by the formula (2) is preferably removed from the polymer from the viewpoint of providing a product of high quality as described above, and thereby it may be possible to avoid the inclusion of PFOA in the final product. One method for preventing these compounds from being contained in the polymer is a technique of removing these compounds in the monomer stage by rectification. However, since the fluoroalkyl alcohol is generally subjected to esterification as a mixture of compounds with "n" of at least zero, the mixture of the alcohols with different "n" values remains in the monomer. Some alcohols may have, depending on "n", boiling points close to that of the fluorine-containing acrylic ester to be used for the polymerization. For this reason, it is difficult to remove all the impurities (that is, a plurality of alcohols) by a simple operation in the monomer phase.

The present invention is made in light of these circumstances, and the object of the present invention is to provide a method for producing the fluorine-containing acrylic esters, which method makes it possible to produce the polymer containing less impurities.

### (Means to Solve the Problem)

The inventors found that the object is achieved by producing fluorine-containing acrylic esters using a mixture of fluoroalkyl alcohols represented by C₂F₅(CF₂CF₂)ₙCH₂CH₂OH which contains a high proportion of the alcohols with "n" of three and four. In other words, the present invention provides a method for producing a mixture of fluorine-containing acrylic esters each of which esters is represented by the formula (1):

C₂F₅(CF₂CF₂)ₙCH₂CH₂OCOCR¹=CH₂ (1)

wherein R¹ is a hydrogen atom, a methyl group or a halogen atom and "n" is an integer of at least zero, which includes:
(A) a step of obtaining a mixture of fluoroalkyl alcohols each of which alcohols is represented by the formula (2):

   C₂F₅(CF₂CF₂)ₙCH₂CH₂OH (2)

   wherein "n" is an integer of at least zero, which mixture contains the fluoroalkyl alcohols with "n" of three(3) and four(4) in an amount of at least 85 mol% in total;
(B) an esterification step of reacting the mixture of fluoroalkyl alcohols obtained in the step (A) with an acrylic acid compound to give a mixture containing the fluorine-containing acrylic esters; and
(C) a step of reducing a proportion of a mixture of the fluoroalkyl alcohols represented by the formula (2) which mixture is contained in the mixture obtained in the step (B).

Herein, the "mixture of fluorine-containing acrylic esters" refers to a mixture containing two or more esters which have different "n" values in the formula (1). The fluorine-containing acrylic ester is generally obtained as such a mixture, but is can be obtained as a compound wherein "n" value is a particular single numeral. However, it seems unlikely that no other esters with other "n" values are contained in the compound when the compound with "n" of the particular single numeral is obtained. For this reason, the term "mixture" is used herein. Similarly, the term "mixture" as to a compound which includes, in its chemical formula, a letter such as "n" or the like corresponding to a polymerization degree is used herein in the sense that it includes a plurality of compounds with different "n" values. Further, in this specification, a symbol "≥" may be used to generically refer to compounds with "n" of "k" or more (or at least "k") and a symbol "≤" may be used to generically refer to compounds with "n" of "k" or less (or at most "k"). Furthermore, a mixture which does not contain a compound with "n" of zero (specifically, the fluorine-containing acrylic ester or the fluoroalkyl alcohol) is identified as a mixture with "n" of one or more and such a mixture can be employed or produced in the present invention.

This production method is characterized in that, as the fluoroalkyl alcohols represented by C₂F₅(CF₂CF₂)ₙCH₂CH₂OCOCR¹OH, ones of n=3 and n=4 are used in the esterification step. For this reason, fluoroalkyl alcohols which do not react in the esterification step and remain in the product are the alcohols with n=3 and n=4 in the above formula (2), that is, C₈F₁₇CH₂CH₂OH and C₁₀F₂₁CH₂CH₂OH. These alcohols have boiling points substantially lower than those of the fluorine-containing acrylic esters and can be separated by rectification. Further, none or, if contained, a small amount of compounds represented by the formula (2) with "n" of five or more (that is, n≥5) exist in the mixture of the fluorine-containing acrylic esters produced according to the method of the present invention. This also contributes to the improvement in purity of the fluorine-containing acrylic esters obtained by the production method according to the present invention. The compounds of n≥5 (particularly, the alcohol represented by the formula (2) with n=5) are difficult to be separated from the fluorine-containing acrylic esters of n=3 and 4 by rectification and tend to remain finally in the polymer as the impurities since the boiling points of the compounds are close to those of the esters of n=3 and n=4. In the production method of the present invention, the impurities in the polymer can be further decreased since such compounds do not exist in the starting materials used in the esterification step.

In the production method of the present invention, the mixture of the fluoroalkyl alcohols represented by C₂F₅(CF₂CF₂)ₙCH₂CH₂OH is prepared which mixture contains the alcohols with n=3 and n=4 in an amount of 85 mol% or more in total, and this mixture is esterified. When the proportion of the sum of the alcohols of n=3 and n=4 is less than 85 mol%, the fluoroalkyl alcohols whose "n" values are small or large are contained in a large amount. The fluorine-containing acrylic esters obtained from the fluoroalkyl alcohols with small "n" values do not form a polymer having good properties as the water- and oil-repellent and therefore it is undesirable that such alcohols are contained in a large amount. As described above, the fluoroalkyl alcohols with large "n" values have boiling points close to those of the fluorine-containing acrylic esters. Therefore, if these alcohols are not esterified and exist (as unreactant) in the ester mixture, they become the impurities which are difficult to be separated from the ester mixture.

The step (A) may be carried out so that only the fluoroalkyl alcohol of n=3 or n=4 can be obtained. However, since C₂F₅(CF₂CF₂)ₙCH₂CH₂OH is usually obtained as a mixture of compounds with different "n" values, complicated operations and equipment are required so as to obtain, from the mixture, another mixture wherein "n" is a particular single integral (for example, a mixture which contains mainly the alcohol of n=3 (or n=4) and the alcohols with other "n" values in only a small amount not to be separated), resulting in economic disadvantage. The production method of the present invention is industrially advantageous in that the elimination of or the reduction in the C₂F₅(CF₂CF₂)ₙCH₂CH₂OH with n≤2 and C₂F₅(CF₂CF₂)ₙCH₂CH₂OH with n≥5 enables the useful fluorine-containing acrylic esters with less impurities to be obtained even if the fluoroalkyl alcohol is used in a form of the mixture.

In the production method of the present invention, the step (A) is preferably carried out as a step of alcohol conversion of a mixture of fluoroalkyl iodides represented by C₂F₅(CF₂CF₂)ₙCH₂CH₂I containing ones with n=3 and n=4 in an amount of 85 mol% or more in total, in which step, for example, the iodides are reacted with an amide and water. The proportion of the total amount of the fluoroalkyl alcohols of n=3 and 4 can be made large in the alcohols after the conversion by subjecting the mixture of the fluoroalkyl iodides containing ones of n=3 and n=4 in a large mount to the alcohol conversion. Alternatively, the step (A) is preferably carried out according to a method wherein the compounds represented by C₂F₅(CF₂CF₂)ₙCH₂CH₂I wherein "n" is an integer of zero or more are subjected to an alcohol conversion to give the mixture of the fluoroalkyl alcohols and then the mixture is subjected to distillation so that the proportions of the fluoroalkyl alcohols with "n" of 2 or less and the fluoroalkyl alcohols with "n" of 5 or more are decreased. In other words, the operation (specifically, distillation) for increasing the proportion of the alcohols with n=3 and n=4 may be carried out either before or after the alcohol conversion.

In the production method of the present invention, the step (C) corresponds to a step of removing, from the mixture obtained in the step (B), the alcohols which are to be impurities and this step corresponds to a step of obtaining a mixture wherein the purity of the esters that are the objective compound is higher. In the production method of the present invention, the step (C) is preferably a distillation step. As described above, since the boiling points of the fluoroalkyl alcohols with n=3 and n=4 are lower than those of the esters that are the objective compound, the distillation is an efficient method for obtaining the mixture with the mixing proportions of these alcohols reduced, and suitable for an industrial mass production.

The present invention provides a method for producing a polymer wherein the mixture of the fluorine-containing acrylic esters produced according to the production method of the present invention are polymerized. Since the starting materials (monomers) with less impurities are used in the method for producing a polymer of the present invention, the resultant polymer also contains less impurities.

### (Effect of Invention)

The production method of the present invention makes it possible to obtain the mixture of the fluorine-containing acrylic esters which contains less impurities. The ester mixture with less impurities improves the quality of the final product (for example, the water- and oil-repellent) which is obtained by polymerizing the esters. Further, the production of PFOA resulting from the impurities can be reduced.

### Embodiments for Carrying Out the Invention

Each step is described below to explain the method of the present invention for producing the mixture of the fluorine-containing acrylic esters.

In the step (A), there is produced the mixture of the fluoroalkyl alcohols represented by C₂F₅(CF₂CF₂)ₙCH₂CH₂OH in which the sum of the proportion of the fluoroalkyl alcohol of n=3 and the proportion of the fluoroalkyl alcohol of n=4 is high. The mixture of C₂F₅(CF₂CF₂)ₙCH₂CH₂OH may be produced by a conventional method. Specifically, the fluoroalkyl alcohols may be produced by, for example, producing the perfluoroalkyl iodides represented by C₂F₅(CF₂CF₂)ₙI by a telomerization reaction wherein C₂F₅I is a telogen and tetrafluoroethylene is a taxogen; adding ethylene to the iodides to give an fluoroalkyl iodides represented by C₂F₅(CF₂CF₂)ₙCH₂CH₂I; and then subjecting this fluoroalkyl iodides to an alcohol conversion reaction. The telomerization reaction and the method of ethylene addition themselves are known and they may be carried out under the conditions usually employed. The alcohol conversion of C₂F₅(CF₂CF₂)ₙCH₂CH₂I itself is known and carried out by, for example, reacting the fluoroalkyl iodide with an amide and water, as described in Japanese Patent Kokoku Publication No. S52-8807(1977). Therefore, the details of those methods are omitted herein. Operations are described herein, which are required for increasing the proportion of the sum of fluoroalkyl alcohols with n=3 and n=4 in the mixture of the alcohols.

The perfluoroalkyl iodide C₂F₅(CF₂CF₂)ₙI is generally obtained as a mixture of compounds with different values of "n" values, as is the case with the telomer obtained by the telomerization reaction. Also the ethylene adduct mixture C₂F₅(CF₂CF₂)ₙCH₂CH₂I which is obtained by subjecting the mixture to ethylene addition is obtained as the mixture of the compound with different "n" values. In the step (A), the compounds with n≤2 and n≥5 need to be removed by conducting, for example, a distillation operation before alcoholification in order to obtain the mixture wherein the fluoroalkyl alcohols with n=3 and n=4 are contained together in an amount of 85 mol% or more. Specifically, any of the following operations is required:
i) The mixture of C₂F₅(CF₂CF₂)ₙI is subjected to distillation to remove the perfluoroalkyl iodides of n≤2 and n≥5; and
ii) The mixture of the ethylene adducts obtained after the ethylene addition is subjected to distillation to remove the fluoroalkyl iodides of n≤2 and n≥5.
   In addition to any of these operations before the alcohol conversion or alternatively, the following operation is required:
iii) The fluoroalkyl alcohols with n≤2 and n≥5 are removed from the mixture of C₂F₅(CF₂CF₂)ₙCH₂CH₂OH which is obtained by carrying out the alcoholification reaction.

The operation i) for obtaining, from the mixtures with n≥0, a mixture in which the proportion of C₂F₅(CF₂CF₂)ₙI with n≤2 and n≥5 is reduced, may be easily conducted by the conventional distillation. Specifically, the desired and intended mixture can be obtained by distilling off the perfluoroalkyl iodides of n≤2 from, for example, the column and withdrawing the perfluoroalkyl iodide of n=3 and n=4 from the side of the rectification column in the distillation operation. In that case, the perfluoroalkyl iodides of n≥5 is withdrawn as bottom or still residue. Alternatively, the desired and intended mixture can be obtained by distilling off the perfluoroalkyl iodides of n≤2 and then withdrawing the perfluoroalkyl iodides of n=3 and n=4 from the column top. Alternatively, the desired and intended mixture may be obtained ay a method wherein a plurality of rectifiers are connected; the perfluoroalkyl iodides with smaller "n" values are removed sequentially; and the perfluoroalkyl iodides of n=3 and n=4 are withdrawn from the final rectifier by being distilled off.

The distillation of the mixture of the perfluoroalkyl iodides is preferably carried out so that the mixture is obtained in which the mixing proportion of the sum of the perfluoroalkyl iodides with n≤2 and n≥5 is less than 15 mol%, and more preferably carried out so that the mixture wherein the proportion of the perfluoroalkyl iodides with n≤2 is less than 13 mol% and the proportion of the perfluoroalkyl iodides with n≥5 is less than 2 mol%. It is preferable that the proportion of the perfluoroalkyl iodides with n≥5 is as low as possible since the alcohols synthesized from the perfluoroalkyl iodides give the compounds which are difficult to be separated from the ester mixture. The specific conditions for distillation (generally, rectification) are the bottom temperature of from 60°C to 140°C, the pressure in the column of from 0.5kPa to 60kPa and theoretical plate number of' from 5 to 25. However, a slight amount of the perfluoroalkyl iodides with n≤2 and n≥5 may exist in the mixture even if any distillation conditions are employed. It should be noted that the preferable proportions of the perfluoroalkyl iodides with n≤2 and n≥5 in the mixture are defined in the above considering such case.

The production of the fluoroalkyl alcohols may be carried out using only the perfluoroalkyl iodide with n=3 or the perfluoroalkyl iodide with n=4. However, since C₂F₅(CF₂CF₂)ₙCH₂CH₂I is usually obtained as the mixture of the compounds having different "n" values, intricate operations and equipment are required in order to obtain the perfluoroalkyl iodide with "n" fixed to one value, which adversely affects economic interest.

The operation ii) is carried out by adding ethylene to the mixture of the perfluoroalkyl iodides obtained by, for example, the telomerization reaction without subjecting the mixture to distillation, and then subjecting the resultant fluoroalkyl iodide mixture C₂F₅(CF₂CF₂)ₙCH₂CH₂I to distillation or the like. The distillation of C₂F₅(CF₂CF₂)ₙCH₂CH₂I is more preferably carried out so that the mixing proportion of the fluoroalkyl iodide of n≤2 is less than 13 mol% and the proportion of the fluoroalkyl iodide of n≥5 is less than 2 mol%. The reason therefor is, as described above in conjunction with the operation i), is to prevent the fluoroalkyl alcohols with n≥5 which are difficult to be separated from the ester mixture from being fed to the step (B). The specific conditions for distillation (generally, rectification) are the bottom temperature of from 60°C to 140°C, the pressure in the column of from 0.5kPa to 2kPa and theoretical plate number of from 10 to 35.

The operation iii) is carried out, in addition to or without the operation of removing the compounds of n≤2 and n≥5 before alcoholification reaction, by subjecting C₂F₅(CF₂CF₂)ₙCH₂CH₂OH obtained by the alcoholification reaction to distillation. The distillation of C₂F₅(CF₂CF₂)ₙCH₂CH₂OH is more preferably carried out so that the mixing proportion of the alcohols with n≤2 is less than 13 mol% and the proportion of the alcohols with n≥5 is less than 2 mol%. The reason therefor is, as described above in conjunction with the operation i), to prevent the fluoroalkyl alcohols with n≥5 which are difficult to be separated from the ester mixture, from being fed to the step (B). The specific conditions for distillation (generally, rectification) are the bottom temperature of from 60°C to 140°C, the pressure in the column of from 0.5kPa to 2kPa and theoretical plate number of from 10 to 35.

The step (B) is a step of reacting the mixture of the alcohols obtained in the step (A) with the acrylic acid compound to obtain the mixture of the fluorine-containing acrylic esters which is the object. The acrylic acid compound may be, for example, acrylic acid, methacrylic acid or 2-halogen-acrylic acid (such as 2-chloroacrylic acid). The step (B) may be carried out under the conditions conventionally employed in an esterification reaction. Specifically, it is carried out using, as a catalyst, a strong acid such as sulfuric acid or p-toluenesulfonic acid at a reaction temperature of from 60°C to 150°C, for example, at 100°C to 130°C. The reaction time is generally 0.1 hours to 10 hours.

As described above, all of the alcohols do not react in the step (B) and the alcohols represented by the formula (2) partially remain as the unreactants. An operation is carried out in the step (C) to obtain the mixture wherein the proportions of these unreactants are lower. Such an operation is, as described above, distillation. The distillation is usually carried out as the rectification when it is conducted industrially. The preferable distillation method employed in the production method of the present invention is described below.

It is preferable that the distillation is conducted so that the mixing ratio of the sum of the fluoroalkyl alcohols represented by the formula (2) with "n" of 0, 1 and 2 is substantially 0 mol%, the mixing ratio of the fluoroalkyl alcohol with "n" of 3 is substantially 0 mol%, the mixing ratio of the fluoroalkyl alcohol with "n" of 4 is from 0 mol% to 0.05 mol%, and the ratio of the sum of the fluoroalkyl alcohols with "n" of 5 or more is from 0 mol% to 0.1 mol%, all the ratios being ones based on the total moles of the fluorine-containing compounds obtained as the distillate. In other words, the distillation is preferably carried out such that the proportion of the fluoroalkyl alcohol occupying the mixture of the fluorine-containing acrylic esters is reduced. Herein, it should be noted that the expression that a component is "substantially 0 mol%" means that the component cannot be detected by usual gas chromatography and the mixture may contain the component in a minute amount within that range. Specifically, such distillation is carried out at the bottom temperature of from 60°C to 160°C and the pressure in the column of from 0.5kPa to 5kPa with the theoretical plate number of from 10 to 35.

It is preferable that the distillation is carried out adding an inhibitor of polymerization to the mixture obtained in the step (C) in order to prevent the fluorine-containing acrylic esters from being polymerized in the distillation column. For example, hydroquinone or hydroquinone monomethyl ether may be added as the inhibitor of polymerization. When these inhibitors are used, it is preferable that the distillation is carried out introducing oxygen or a gas containing oxygen (for example, air) into the distillation column. The introduction of oxygen further suppresses the polymerization of the fluorine-containing acrylic esters. The inhibitor of polymerization used in the step (C) is not necessarily required to be hydroquinone or hydroquinone monomethyl ether and may be another one.

Further, a resin lining is preferably used in the distillation column. Furthermore, a resin packing is preferably packed in the distillation column. Metal is excluded from the interior of the distillation column by forming the members inside the distillation column of a resin as described above. As a result, the deterioration of the inhibitor of polymerization is effectively prevented, and therefore the polymerization of the fluorine-containing acrylic esters can be more effectively prevented during the distillation. Alternatively, the lining or the packing may be formed of a metal having a high electrode potential.

During the distillation, the alcohols represented by the formula (2) are withdrawn from the top of the distillation column and then the desired mixture of fluorine-containing acrylic esters is withdrawn from the column top or as the still residue or the bottom. The most of the alcohols distilled off is from C₂F₅(CF₂CF₂)ₙCH₂CH₂OH with n=3 and n=4 and the boiling points of these alcohols are low. Therefore, the mixture of the fluorine-containing acrylic esters with a high purity can be obtained even if the bottom temperature falls in the low range as described above.

Contrariwise, when the fluoroalkyl alcohols of n≥5 is not sufficiently reduced, the fluoroalkyl alcohol of n=5 is contained in the mixture to be subjected to the distillation step in the step (C). In that case, there is a disadvantage that the theoretical plate number needs to be a large one in order to remove the fluoroalkyl alcohol of n=5 since the boiling point of this fluoroalkyl alcohol is particularly close to those of the fluorine-containing acrylic esters of n=3 and 4. Further, the bottom temperature is required to be high in order to withdraw, as the objective compound, the fluorine-containing acrylic esters of n≥5 resulting from the fluoroalkyl alcohols n≥5 together with the esters of n=3 and 4 because the esters of n≥5 have high boiling points. When the bottom temperature is high, the polymerization is promoted and therefore the inhibitor of polymerization needs to be added in a larger amount. The larger amount of the polymerization inhibitor added causes disadvantage that the impurities derived from the inhibitor are increased. For this reason, the use of the fluoroalkyl alcohols of n≥5 is not desirable from the viewpoints of the efficiency of the distillation step after the step (A) and the quality of the intended mixture obtained after the distillation step. The step (C) is preferably conducted as the distillation step, but this step is not limited to the distillation.

A fluorine-containing acrylate-based polymer can be produced by subjecting the mixture obtained in the step (C) to the polymerization step. The polymerization may be carried out employing any polymerization condition which is conventionally employed. The resultant polymer has excellent quality since it contains less impurities. The resultant polymer is useful as the water- and oil-repellent for treating a surface of a substrate, such as textile products, stone material, a filter (for example, an electrostatic filter), a dust-protective mask, a fuel battery, glass, paper, wood, leather, fur skin, asbestos, brick, cement, metal and oxide, ceramic material, and plastics. Further, the resultant polymer is useful as a water- and oil-repellent and antifouling finish for carpeting.

### Examples

### (Example 1)

A mixture of fluoroalkyl alcohols represented by C₂F₅(CF₂CF₂)ₙCH₂CH₂OH containing one with n=3 in an amount of 80 mol% and one with n=4 in an amount of 20 mol% was produced by a technique of alcoholification of a mixture of C₂F₅(CF₂CF₂)ₙCH₂CH₂I. 150g (310mmol) of this fluoroalkyl alcohol mixture, 22.3g (260mmol) of acrylic acid, 50g of toluene, 1.48g (7.78mmol) of p-toluenesulfonic acid and 0.05g of hydroquinone were charged into a 300mL glass reactor equipped with a stirrer and subjected to esterification reaction for 4 hours while distilling off water, to give the reaction mixture having a composition as shown in Table 1. This reaction mixture was charged into a still and subjected to distillation using a rectifier with a theoretical plate number of ten(10) and selecting a pressure inside the rectifier and a still temperature within a range of from 0.3 kPa to 15 kPa and a range of from 100°C to 160°C respectively depending on compounds to be distilled off, so that toluene, acrylic acid, unreacted alcohols, the fluorine-containing acrylic esters (n=3 and n=4) and substances of high boiling points were distilled off in this order. As a result, the mixture after distillation was given as the distillate which was obtained by cutting toluene, acrylic acid and the unreacted alcohols as an initial distillate and the substances of high boiling points as a final distillate, and thereby contained the fluorine-containing acrylic esters as a main component and had a composition as shown in Table 1. In Table 1, the compositions of the reaction mixture and the mixture after distillation were determined by gas chromatography.

**Table 1**

| Component | Reaction mixture (excluding toluene) (mol%) | Mixture after distillation (mol%) |
|---|---|---|
| C₈F₁₇CH₂CH₂OH | 2.19 | ND |
| C₁₀F₂₁CH₂CH₂OH | 0.65 | 0.03 |
| C₈F₁₇CH₂CH₂OCOCH=CH₂ | 74.93 | 79.81 |
| C₁₀F₂₁CH₂CH₂OCOCH=CH₂ | 18.60 | 19.50 |
| Other substances of high boiling points | 3.63 | ND |

As shown in Table 1, of the impurities contained in the reaction mixture, C₈F₁₇CH₂CH₂OH (n=3) and the substances of high boiling points were able to be removed by the distillation. As a result, the mixture of the fluorine-containing acrylic esters with a high purity was obtained. Specifically, only C₁₀F₂₁CH₂CH₂OH (n=4) was contained as the impurity in an amount of 0.03 mol%. In this example, an yield of the fluorine-containing acrylic esters was 85 mass%.

### (Example 2)

The reaction mixture having a composition shown in Table 2 was obtained in the same manner as that in Example 1 except that the amount of acrylic acid used for the esterification reaction was changed to 18.7g, and then the distillation was carried out in the same manner as that in Example 1 to obtain the mixture after distillation having a composition as shown in Table 2 as the distillate liquid containing the fluorine-containing acrylic esters as the main component. In Table 2, the compositions of the reaction mixture and the mixture after distillation were determined by gas chromatography.

**Table 2**

| Component | Reaction mixture (excluding toluene) (mol%) | Mixture after distillation (mol%) |
|---|---|---|
| C₈F₁₇CH₂CH₂OH | 12.3 | ND |
| C₁₀F₂₁CH₂CH₂OH | 3.3 | 0.04 |
| C₈F₁₇CH₂CH₂OCOCH=CH₂ | 65.8 | 79.95 |
| C₁₀F₂₁CH₂CH₂OCOCH=CH₂ | 16.8 | 20.01 |
| Other substances of high boiling points | 2.1 | ND |

As shown in Table 2, although the reaction mixture obtained in Example 2 contained more unreacted alcohols compared with that obtained in Example 1, the highly-pure mixture of the fluorine-containing acrylic esters with less impurities was obtained by the distillation. Specifically, only C₁₀F₂₁CH₂CH₂OH (n=4) was contained in an amount of 0.04 mol% as the impurity. In this example, an yield of the fluorine-containing acrylic esters was 75 mass%.

### (Example 3)

The reaction mixture having a composition as shown in Table 3 was obtained in the same manner as that in Example 1 except that 166.1g (310 mmol) of the mixture which had a composition as shown in Table 3 was used as the mixture of the fluoroalkyl alcohols acrylic acid. Next, this reaction mixture was charged into the still and subjected to distillation using a rectifier with the theoretical plate number of ten(10) and selecting the pressure inside the rectifier and the still temperature within a range of from 0.3 kPa to 15 kPa and a range of from 100°C to 160°C respectively depending on compounds to be distilled off, so that toluene, acrylic acid, unreacted alcohols, the fluorine-containing acrylic esters (3≤n≤7) and substances of high boiling points were distilled off in this order. As a result, the mixture after distillation was given as the distillate which was obtained by cutting toluene, acrylic acid and the unreacted alcohols as the initial distillate and the substances of high boiling points as the final distillate, and thereby contained the fluorine-containing acrylic esters as the main component and had a composition shown in Table 3. In Table 3, the compositions of the reaction mixture and the mixture after distillation were determined by gas chromatography.

**Table 3**

| Component | Mixture of fluoroalkyl alcohols (mol%) | Reaction mixture (excluding toluene) (mol%) | Mixture after distillation (mol%) |
|---|---|---|---|
| C₈F₁₇CH₂CH₂OH | 84.79 | 3.90 | ND |
| C₁₀F₂₁CH₂CH₂OH | 12.92 | 0.54 | 0.03 |
| C₁₂F₂₅CH₂CH₂OH | 1.94 | 0.04 | 0.04 |
| C₁₄F₂₉CH₂CH₂OH | 0.31 | 0.01 | 0.01 |
| C₁₆F₃₃CH₂CH₂OH | 0.04 | ND | ND |
| C₈F₁₇CH₂CH₂OCOCH=CH₂ | | 77.89 | 83.28 |
| C₁₀F₂₁CH₂CH2OCOCH=CH₂ | | 12.00 | 13.04 |
| C₁₂F₂₅CH₂CH₂OCOCH=CH₂ | | 1.71 | 1.89 |
| C₁₄F₂₉CH₂CH₂OCOCH=CH₂ | | 0.26 | 0.28 |
| C₁₆F₃₃CH₂CH₂OCOCH=CH₂ | | 0.04 | 0.05 |
| Other substances of high boiling points | | 3.58 | 1.38 |

In Example 3, the unreacted alcohols of n≥5 remained in the resultant reaction mixture because the mixture containing the fluoroalkyl alcohols of n≥5 was esterified. Although most of the alcohols of n≥5 were not able to be removed, the highly-pure mixture of the fluorine-containing acrylic esters was obtained in which the impurity content was small as a whole because most of the alcohols of n≤4 were able to be removed. Specifically, the mixture contained, as the impurities, C₁₀F₂₁CH₂CH₂OH (n=4) in an amount of 0.03 mol%, C₁₂F₂₅CH₂CH₂OH (n=5) in an amount of 0.04 mol%, C₁₄F₂₉CH₂CH₂OH (n=6) in an amount of 0.01 mol% and the impurities of high boiling points in an amount of 1.38%. In this example, a yield of the fluorine-containing acrylic esters was 84 mass%.

### (Comparative Example 1)

The reaction mixture having a composition as shown in Table 4 was obtained in the same manner as that in Example 1 except that 178.6g (310 mmol) of the mixture which had a composition as shown in Table 4 was used as the mixture of the fluoroalkyl alcohols. Next, this reaction mixture was charged into the still and subjected to rectification in the same manner as that in Example 1 to obtain the mixture after distillation as a distillate liquid which contained the fluorine-containing acrylic esters as the main component and had a composition shown in Table 4. In Table 4, the compositions of the reaction mixture and the mixture after distillation were determined by gas chromatography.

**Table 4**

| Component | Mixture of fluoroalkyl alcohols (mol%) | Reaction mixture (excludin g toluene) (mol%) | Mixture after distillation (mol%) |
|---|---|---|---|
| C₈F₁₇CH₂CH₂OH | 60.55 | 2.57 | ND |
| C₁₀F₂₁CH₂CH₂OH | 23.97 | 1.55 | 0.09 |
| C₁₂F₂₅CH₂CH₂OH | 9.94 | 0.79 | 0.83 |
| C₁₄F₂₉CH₂CH₂OH | 3.22 | 0.30 | 0.32 |
| C₁₆F₃₃CH₂CH₂OH | 1.92 | 0.07 | 0.07 |
| C₁₈F₃₇CH₂CH₂OH | 0.40 | ND | ND |
| C₈F₁₇CH₂CH₂OCOCH=CH₂ | | 56.26 | 59.27 |
| C₁₀F₂₁CH₂CH₂OCOCH=CH₂ | | 22.11 | 23.51 |
| C₁₂F₂₅CH₂CH₂OCOCH=CH₂ | | 8.18 | 8.53 |
| C₁₄F₂₉CH₂CH₂OCOCH=CH₂ | | 2.51 | 2.57 |
| C₁₆F₃₃CH₂CH₂OCOCH=CH₂ | | 1.59 | 1.60 |
| C₁₈F₃₇CH₂CH₂OCOCH=CH₂ | | 0.28 | 0.27 |
| Other substances of high boiling point | | 3.79 | 2.94 |

In Comparative Example 1, the proportion of C₁₀F₂₁CH₂CH₂OH (n=5) was high, 0.83 mol%, and the total proportion of the impurities was high, 17.22 mol% in the mixture after distillation because the mixture containing the alcohols with n≥5 at a high proportion was used. In Comparative Example 1, a yield of the fluorine-containing acrylic esters was 78 mass%.

As described above, the mixture of the fluorine-containing acrylic esters can be obtained wherein the proportion of the impurities is reduced to a ppm level according to the production method of the present invention. The monomer mixture with less impurities is useful for producing a polymer of high quality. Further, the method of the present invention makes it possible to give the mixture which contains the fluorine-containing acrylic esters with "n" values of 3 and 4 at a high ratio and the fluorine-containing acrylic esters with n≤2 or n≥5 at a low ratio. As described above, the fluorine-containing acrylic esters with n=3 and 4 are monomers which produce the polymer useful as the water- and oil-repellent, and therefore the production method of the present invention contributes to improvement in the quality of the polymer in this point.

### Industrial Applicability

The production method of the present invention makes it possible to obtain the mixture of the fluorine-containing acrylic esters represented by the formula C₂F₅(CF₂CF₂)ₙCH₂CH₂OCOCR¹=CH₂ which mixture contains a high proportion of the esters of n=3 and n=4 and a low proportion of the other compounds. Therefore, the mixture of the fluorine-containing acrylic esters obtained by this production method is suitable for being used as the monomer for producing the polymer which is particularly useful as the water- and oil-repellent.

## Claims

1. A method for producing a mixture of fluorine-containing acrylic esters each of which esters is represented by a formula (1):
C₂F₅(CF₂CF₂)ₙCH₂CH₂OCOCR¹=CH₂ (1)
wherein R¹ is a hydrogen atom, a methyl group or a halogen atom and "n" is an integer of at least zero, which comprises:
(A) a step of obtaining a mixture of fluoroalkyl alcohols each of which alcohols is represented by a formula (2):
C₂F₅(CF₂CF₂)ₙCH₂CH₂OH (2)
wherein "n" is an integer of at least zero, which mixture contains the fluoroalkyl alcohols with n=3 and n=4 in an amount of at least 85 mol% in total;
(B) an esterification step of reacting the mixture of fluoroalkyl alcohols obtained in the step (A) with an acrylic acid compound to give a mixture containing the fluorine-containing acrylic esters; and
(C) a step of reducing a proportion of a mixture of the fluoroalkyl alcohols each of which is represented by the formula (2) which mixture is contained in the mixture obtained in the step (B).

2. The method according to claim 1 wherein a proportion of the fluoroalkyl alcohols represented by the formula (2) with "n" of 0, 1 and 2 is less than 12 mol% and a proportion of the fluoroalkyl alcohols with "n" of 5 or more is less than 3 mol% in the mixture obtained in the step (A).

3. The method according to claim 1, wherein the step (C) is carried out by subjecting the mixture obtained in the step (B) to distillation.

4. The method according to claim 3, wherein the distillation is conducted so that a proportion of the fluoroalkyl alcohols represented by the formula (2) with "n" of 0, 1 and 2 is substantially 0 mol%, a proportion of the fluoroalkyl alcohol with "n" of 3 is substantially 0 mol%, a proportion of the fluoroalkyl alcohol with "n" of 4 is from 0 mol% to 0.05 mol%, and a proportion of the fluoroalkyl alcohols with "n" of 5 or more is from 0 mol% to 0.1 mol%, based on the total moles obtained as distillate in the step (C).

5. The method according to claim 1, wherein the step (A) is carried out by converting a mixture of compounds to alcohols wherein:
each of the compounds is represented by C₂F₅(CF₂CF₂)ₙCH₂CH₂I wherein "n" is an integer of at least zero; and
the mixture contains the compounds with n=3 and n=4 in an amount of 85 mol% or more in total.

6. The method according to claim 1, wherein the step (A) is carried out by subjecting a mixture of the fluoroalkyl alcohols to distillation to reduce proportions of the fluoroalkyl alcohols with n≤2 and n≥5, wherein the fluoroalkyl alcohols is obtained by converting compounds to the fluoroalkyl alcohols each of which compounds is represented by C₂F₅(CF₂CF₂)ₙCH₂CH₂I wherein "n" is an integer of at least 0.

7. A method for producing a polymer of fluorine-containing acrylic esters each of which is represented by a formula (1) :
C₂F₅(CF₂CF₂)ₙCH₂CH₂OCOCR¹=CH₂ (1)
wherein R¹ is a hydrogen atom, a methyl group or a halogen atom and "n" is an integer of at least zero, which comprises:
(A) a step of obtaining a mixture of fluoroalkyl alcohols each of which alcohols is represented by a formula (2):
C₂F₅(CF₂CF₂)ₙCH₂CH₂OH (2)
wherein "n" is an integer of at least zero, which mixture contains the fluoroalkyl alcohols with n=3 and n=4 in an amount of at least 85 mol% in total;
(B) a step of reacting the mixture of fluoroalkyl alcohols obtained in the step (A) with an acrylic acid compound to give a mixture containing the fluorine-containing acrylic esters;
(C) a step of reducing a proportion of a mixture of the fluoroalkyl alcohols each of which is represented by the formula (2) which mixture is contained in the mixture obtained in the step (B); and
(D) polymerizing a mixture obtained in the step (C).

8. A fluorine-containing acrylate polymer produced by the method according to claim 7.
